# FASCICULE DE BREVET EUROPEEN

(11) **EP 0 983 216 B1**
(45) Date de publication et mention de la délivrance du brevet: **02.08.2006**
(21) Numéro de dépôt: 98925721.7
(22) Date de dépôt: 15.05.1998
(51) Int. Cl.: C07B 45/00, C07C 303/38, C07C 311/48

(54) **PROCEDE DE SYNTHESE DE PERFLUOROSULFONAMIDES, DE PERFLUOROSULFONAMIDES ET DE LEURS SELS, AINSI QU'UN REACTIF DE SULFONYLATON**
VERFAHREN ZUR HERSTELLUNG VON PERFLUORSULFONAMIDEN, PERFLUORSULFONAMIDEN UND IHREN SALZEN, SOWIE EIN SULFONIERUNGSREAGENS
METHOD FOR THE SYNTHESIS OF PERFLUOROSULPHONAMIDES, PERFLUOROSULPHONAMIDES AND THEIR SALTS, AND A SULPHONATION REAGENT

(30) Priorité: 16.05.1997 FR 9706064
(43) Date de publication de la demande: 08.03.2000
(73) Titulaire: RHODIA CHIMIE, 92408 Courbevoie Cédex (FR)
(72) Inventeur: PEVERE, Virginie, F-69008 Lyon (FR); MARX, Emmanuel, F-69380 Châtillon d'Azergues (FR); GILBERT, Laurent, F-69007 Lyon (FR)
(74) Mandataire: Dutruc-Rosset, Marie-Claude
(86) Numéro de dépôt international: PCT/FR1998/000973
(87) Numéro de publication internationale: WO 1998/052886

(56) Documents cités:
- WO-A-90/11999
- WO-A-97/23448
- FR-A- 2 724 380
- L.M. YAGUPOL'SKII, ET AL.: "Trifluoromethylsulphonylimino and bis(trifluoromethylsulphonylimino) derivatives of arenesulphonic acids" RUSSIAN JOURNAL OF ORGANIC CHEMISTRY, vol. 31, no. 5, mai 1995, pages 691-695, XP002051637 New York, US
- D.D. DESMARTEAU, ET AL.: "N-Fluoro-bis(trifluoromethanesulphonyl)im ide. An improved synthesis" JOURNAL OF FLUORINE CHEMISTRY, vol. 52, no. 1, avril 1991, pages 7-12, XP002025807 LAUSANNE, CH
- B. HELFERICH, ET AL.: "Bis-[alkylsulfonsäure]-imide" BERICHTE DER DEUTSCHEN CHEMISCHEN GESELLSCHAFT, vol. 75, 1942, pages 532-536, XP002025808 WEINHEIM, DE

## Description

La présente invention a pour objet un procédé de synthèse de perfluorosulfonamides et de perfluorosulfonimides. Ces derniers peuvent être obtenus sous la forme de leurs sels. La présente invention vise aussi un réactif de perfluorosulfonylation.

Elle concerne plus particulièrement une réaction de sulfonation de fonction azotée porteuse d'un radical électroattracteur. Elle vise aussi particulièrement la sulfonation d'un sulfonamide notamment fluoré. La synthèse de sulfamides vise surtout le cas où le sulfamide est préparé dans le but d'une sulfonation ultérieure (dans une deuxième étape, dans le même réacteur, ou simultanément in situ).

Les dérivés fluorés de sulfonimides sont de plus en plus développés pour des applications électriques et notamment pour la constitution de piles et pour leur vertu catalytique. Le composé dérivé de ces sulfonimides le plus fréquemment utilisé est le dérivé lithié, c'est-à-dire le sel de cet imide, imide qui est lui-même très acide.

La synthèse de ces sulfonimides a été déjà réalisée mais met en oeuvre des procédés particulièrement délicats et difficiles à utiliser.

La demande intercalaire WO 97/23.448 décrit un procédé qui, comme tous ceux qui l'ont précédé (Cf. FR-A-2.724.380), utilise des fluorures de perfluoroalcanesulfonyle, dont la réactivité est très spécifique et dont le sous-produit est l'ion fluorure qui nécessite l'emploi de matériaux qui y résiste, et donc coûteux, ainsi qu'une élimination poussée avant le rejet des effluents. La très forte volatilité et les conditions opératoires conduisent dans les cas les plus courants à travailler sous des pressions relativement élevées. Brochant sur le tout, la sensibilité à l'eau des fluorures de perfluoroalcanesulfonyle semble, au vu de ce document particulièrement élevée.

Quelques essais ont été réalisés sur des halogénures de sulfonyle mais ont conduit à des échecs (sur les problèmes relatifs à ces synthèses voir la demande WO 90/11.999, en particulier page deux, lignes 30 à 35 et passim). C'est notamment le cas du bis trifluorométhylsulfinimide qui semble particulièrement difficile à réaliser à partir de chlorure de trifluorométhanesulfonyle. C'est pourquoi un des buts de la présente invention est de fournir un procédé qui permette d'obtenir des imides fluorés du type précédent en utilisant des halogénures de sulfonyle lourds (c'est-à-dire des halogénures correspondant à un halogène de nombre atomique au moins égal à celui du chlore).

On préfère utiliser pour des raisons économiques les chlorures de sulfonyle. Ce but et d'autres qui apparaîtront par la suite sont atteints au moyen d'un procédé de synthèse qui comprend une étape de mise en contact d'un nucléophile dont l'atome nucléophile est un azote, avec un réactif comportant pour addition successive ou simultanée :
chlorure de sulfonyle; et
une base organique à la fois non alcoylable et liposoluble ;
par le fait que la partie organique dudit sulfonyle est perfluorée sur le carbone porté par le soufre et par le fait que ladite base organique à la fois non alcoylable et liposoluble est choisi pami les parmi les dialcoylphosphines encombrées, trialcoylphosphines, les hydroxydes de phosphonium, les dialcoylamines encombrées, trialcoylamines, les hydroxydes d'ammonium, avec la condition que lorsque quand la réaction est menée sans un solvant, ce dernier est choisi parmi ceux dont la polarité (E^{f}ₜ exprimée en kcal par mol) est au plus égale à 40 et avec la condition que quand la réaction est menée sans solvant, les bases organiques sont choisies parmi les trialcoylamines de nombre de carbones superieur à 6 ; les trialcoylamines de nombre de carbone inférieur à 7, mais présentant au moins un radical secondaire ou tertiaire, avantageusement deux.

Ainsi la présente invention fournit un procédé de synthèse de perfluorosulfonamides, et de sulfonimides qui présente au moins un, avantageusement deux, groupe sulfonyle dont le carbone jouxtant le soufre est perfluoré.

Les sels de ces imides, se préparent, de manière en elle-même connue, à partir de ces imides, le procédé selon la présente invention peut comporter une étape de préparation de ces sels (comme dans les exemples).

La notion de base organique à la fois non alcoylable et liposoluble est explicitée par la suite.

Ledit azote de la fonction nucléophile est avantageusement porteur d'un hydrogène ou d'une charge négative (anion).

Il est préféré que ledit azote de la fonction nucléophile soit porteur de deux hydrogènes (et même 3 pour le cas particulier de l'ammoniac, ou de l'ammoniaque, qui permette de synthétiser la catégorie de substrats préférée, à savoir les sulfamides, avantageusement perfluorés sur le carbone jouxtant le soufre, de préférence répondant à la définition de Rf) ou bien d'un hydrogène et d'une charge négative (anion).

En particulier le nucléophile peut être un sulfonamide (sulfamide) notamment sous la forme d'un sel, avantageusement de base organique non alcoylable. Le procédé convient en particulier même lorsque la partie organique dudit sulfonamide (sulfamide) est perfluorée sur le carbone porté par le soufre.

Les sulfamides usuels (correspondant à des acides sulfoniques non perfluorés, tels que Ar-SO₃H avec Ar représentant un aryle et RSO₃H avec R représentant un alcoyle) ne justifie pas d'être sous forme de sel, sel qui d'ailleurs serait difficile à obtenir avec les bases préférées de l'invention. Dans la présente description ALCO-***yle*** est pris dans son sens étymologique de reste hydrocarboné d'un ALCO-***ol*** après ignorance de la fonction alcool (ou ol).

Le substrat nucléophile présente ainsi comme fonction nucléophile, un fonction avantageusement choisie parmi les sulfamides des acides sulfoniques dont le soufre est lié à un aryle ou un reste aliphatique y compris les alcoyles, de préférence à un reste aliphatique perfluorés sur le carbone jouxtant le soufre. En général le nombre de carbone du substrat nucléophile varie de 1 à 15, et même de 1 à 10.

Ainsi, de ce qui précède et de l'étude qui a mené à la présente invention, il appert que les nucléophiles (sous forme neutre ou surtout d'anion) dont les problèmes, car difficiles, sont particulièrement bien résolus par la présente invention sont ceux dont l'acide associé présente un pKa au plus égal à environ 7, avantageusement à 6, de préférence à 5.

L'ammoniac constitue un cas à part et peut, dans le cas de l'ammoniac conduire à un imide par deux condensations successive in situ.

La condensation des halogénures lourds de sulfonyle visés par la présente invention sur l'ammoniac, ou l'ammoniaque, peut constituer une étape préalable à la condensation des amides.

Dans la présente description le terme "environ" est employé pour mettre en exergue le fait que les valeurs qui le suivent correspondent à des arrondis mathématiques et notamment que lorsque le, ou les, chiffres les plus à droite d'un nombre sont des zéros, ces zéros sont des zéros de position et non des chiffres significatifs, sauf bien entendu s'il en est précisé autrement..

Avantageusement ladite base organique non alcoylable est choisie parmi les dialcoylphosphines encombrées, trialcoylphosphines, les hydroxydes de phosphonium, les dialcoylamines encombrées, trialcoylamines, les hydroxydes d'ammonium. On peut également envisager les cycles phosphorés et azotés présentant une basicité adéquate (voir infra).

Il est souhaitable que ladite base à la fois non alcoylable et liposoluble soit choisie parmi les dialcoylphosphines encombrées, trialcoylphosphines, les hydroxydes de phosphonium, les dialcoylamines encombrées, trialcoylamines, les hydroxydes d'ammonium.

Il est conseillé que ladite base à la fois non alcoylable et liposoluble présente au moins une solubilité dans le benzène significative (symbole "s" dans le "handbook of chemistry and physics"), avantageusement (symbole "v" dans le "handbook of chemistry and physics") élevée.

Il est conseillé que le substrat présente au moins une solubilité dans le benzène significative (symbole "s" dans le "handbook of chemistry and physics"), avantageusement (symbole "v" dans le "handbook of chemistry and physics") élevée.

De même, selon la présente invention, on préfère faire en sorte que le substrat nucléophile soit sous la forme d'un sel de base organique non alcoylable, cet éventuel composé salin entre la base organique et ledit substrat (lorsque il est acide comme dans le cas du perfluorosulfonamide) présente au moins une solubilité dans le benzène significative (symbole "s" dans le "handbook of chemistry and physics"), avantageusement (symbole "v" dans le "handbook of chemistry and physics") élevée.

Dans les cas précédents, il est bien sûr particulièrement satisfaisant que la solubilité soit telle que le benzène et les bases visées ci-dessus soient miscibles en toute proportion (symbole "∞" dans le "handbook of chemistry and physics").

Pour choisir la base, il convient de respecter des contraintes de basicité, ainsi il est souhaitable que le pKa de l'acide associé à ladite base organique (formant l'éventuel sel avec le nucléophile) soit supérieur à ou voisin de celui dudit nucléophile [par exemple sulfonamide (lequel est porteur en principe de deux hydrogènes sur l'azote)].

De même, il est également souhaitable que le pKa de l'acide associé à ladite base non alcoylable et liposoluble soit égal et de préférence supérieur à celui dudit sulfonamide.

Lorsqu'il y a inégalité, il est souhaitable que la différence entre les pKa des acides associés et celui dudit nucléophile (soit au moins égale à 1, avantageusement à 2, de préférence à 3.

Quoique que cela ne soit pas préféré les bases peuvent être des mélanges de bases à condition quel le mélange réponde aux contraintes et, même préférence, spécifiée ci dessus.

On peut préciser que lorsque ledit nucléophile est sulfamide, pour des raisons de facilité de manipulation, il est préférable que ladite base organique non alcoylable et ladite base à la fois non alcoylable et liposoluble soient identiques. La réaction peut être menée sans solvant, notamment lorsque les bases organiques sont choisies parmi les préférées, c'est-à-dire particulièrement liposolubles et peu polaires (par exemple et notamment, trialcoylamines de nombre de carbone supérieur à 6 ; trialcoylamines de nombre de carbone inférieur à 7, mais présentant au moins un radical secondaire ou tertiaire, avantageusement deux, ou dialcoylamine encombrée).

En ce qui concerne une mise en oeuvre avantageuse de ladite étape, la mise en contact est réalisée dans un solvant organique, avantageusement peu polaire, de préférence peu miscible à l'eau (au plus 10% en masse, avantageusement au plus 5% en masse, de préférence au plus 2% en masse).

La température de réaction est avantageusement au moins égale au point de fusion finissante (hors insolubles et notamment sels [halohydrate,..]) du mélange réactionnel et avantageusement au plus 100°C (avantageusement deux chiffres significatif, de préférence 3), avantageusement à une température au plus égale à environ 50°C, de préférence à 40°C et avantageusement au moins égale à 0. Ainsi la température réactionnelle est avantageusement située dans l'intervalle fermé défini par le point de fusion finissante et 100°C. De préférence dans l'intervalle 0°C et 50°C, plus préférentiellement dans l'intervalle 0 et 40°C. Quoique la réaction puisse être menée sous une pression différente, il est plus aisé de la mener sous pression atmosphérique. Dans ce cas il peut être opportun de choisir les solvants de manière qu'il y ait un reflux possible à une température choisie dans les domaines ci-dessus de température.

Pour obtenir une bonne cinétique de réaction, il est préconisé de réaliser ladite mise en contact dans un solvant organique lequel est choisi de manière que lorsque ledit nucléophile est sel de sulfamide, ledit sel y soit soluble, avantageusement à hauteur d'une concentration d'au moins 0,05 M, de préférence d'au moins 0,2 M.

Pour obtenir les solubilités ci-dessus on peut jouer aussi sur ladite base organique non alcoylable. On peut également jouer sur le solvant et la base organique simultanément.

Les bases mises en oeuvre dans la présente invention présentent avantageusement de 3 à environ 40 atomes de carbone, de préférence de 6 à environ 30 atomes de carbone, plus préférentiellement de 8 à 25 atomes de carbone. Notamment lorsque le nombre de carbone est faible (c'est-à-dire moins de 7), il est préférable que l'un au moins des substituants de l'atome basique soit au moins secondaire. Ces bases sont, pour des raisons économiques, avantageusement des amines.

Les bases peuvent être également des bases polyfonctionnelles (par exemple les éthylènediamines substituées et notamment la tétraméthyléthylènediamine), auquel cas la contrainte ci-dessus doit être ramenée au nombre de fonctions basiques utiles.

Les solvants les plus utiles sont des solvants relativement peu polaires du type solvants chlorés ou solvants aromatiques à condition préférentielle que ledit éventuel sel y soit suffisamment soluble.

Ledit solvant peu polaire qui, rappelons-le, peut être un mélange, est avantageusement choisi parmi ceux dont la polarité (E^{f}ₜ exprimée en kcal par mol) est au plus égale à 40 (avantageusement deux chiffres significatifs). Toutefois pour des raisons d'hygiène du travail et de protection de l'environnement [certains sont d'ores et déjà interdits], les dérivés chlorés non aromatiques, notamment aliphatiques (tel que chlorure de méthylène ; chloroforme) ou alcènique (Par exemple trichloréthylène) sont, en général, à éviter comme solvant. Par ailleurs, quoique donnant de bons résultats, ils ne constituent pas la famille la plus performante (ainsi la solubilité dans l'eau du chlorure de méthylène est de l'ordre 2% en volume, soit 2,6% en masse).

Compte tenu de ce qui précède, ledit solvant peu polaire est avantageusement choisi parmi les composés organiques oxygénés, (notamment les éthers, les esters, voire les cétones), les hydrocarbures (y compris fractions pétrolières), les hydrocarbures aromatiques halogénés sur le noyau.

De préférence, l'on peut avantageusement choisir ledit solvant peu polaire parmi les benzènes substitués et les hydrocarbures halogénés sur le noyau.

Le respect de la stoechiométrie de la réaction est souhaitable en ce qui concerne l'halogénure de sulfonyle et le sulfonamide (sulfamide). Une tolérance de plus ou moins 20% est tout à fait acceptable et dépend essentiellement du coût respectif des réactifs.

En ce qui la concerne, la quantité de ladite base non alcoylable et liposoluble mise en jeu au cours de la réaction, est au moins égale à la quantité nécessaire pour neutraliser l'acide halohydrique dégagé.

Lorsque l'azote du substrat porte deux hydrogènes (trois dans le cas très particulier de l'ammoniac, qui permet de synthétiser, notamment in situ, la catégorie de substrats préférée, à savoir les sulfamides, avantageusement perfluorés sur le carbone jouxtant le soufre, de préférence répondant à la définition de Rf ; bien entendu dans le cas d'une synthèse in situ il faut tenir compte de la réaction de sulfonylation de l'ammoniac), et que le nucléophile n'est pas sous forme de sel, il peut être intéressant de porter la quantité de base(s) mise en jeu au cours de la réaction à une valeur au moins égale à deux fois la quantité nécessaire pour neutraliser l'acide halohydrique dégagé.

En d'autres termes et plus précisément, il est préféré que la somme des bases (base du sel substrat [voir supra] et base organique liposoluble non alcoylable) soit au moins égale à la somme de l'acidité dégagée sous forme d'acide halohydrique et de l'acidité des composés sulfonés (surtout sulfonimides) en cours de formation.

Ainsi compte tenu de ce qui précède, il est souhaitable que la quantité totale de base(s) soit au moins égale à une fois quantité stoechiométriquement nécessaire pour neutraliser l'acide halohydrique dégagé, avantageusement au moins égale à la somme de l'acidité dégagée sous forme d'acide halohydrique et de l'acidité des composés sulfonés (surtout sulfonimides); plus spécifiquement II est préconisé d'utiliser une quantité de base (exprimée bien sûr en équivalent) au moins égale à la somme de l'acidité des composés sulfonés et de une fois et quart la quantité stoechiométriquement nécessaire pour neutraliser l'acide halohydrique dégagé, avantageusement au moins égale à la somme de une fois et demie l'acidité dégagée sous forme d'acide halohydrique et de l'acidité des composés sulfonés.

Il est également préférable d'éviter un excès trop important de base(s) ; c'est pourquoi Il est souhaitable que la somme des bases (base du sel substrat [voir supra] et base organique liposoluble non alcoylable) présente un excès par rapport à la somme de l'acidité dégagée sous forme d'acide halohydrique et de l'acidité des composés sulfonés en cours de formation au plus égale à 3 fois, avantageusement à deux fois, de préférence à une fois la quantité d'acide halohydrique dégagé.

Le procédé sur la présente invention est particulièrement intéressant pour la synthèse des composés obtenus à partir de chlorure de sulfonyle dont la partie organique est perfluorée (c'est-à-dire correspond à CX₂, voir infra) sur le carbone porté par le soufre.

La présente invention vise en particulier le cas où la partie organique dudit chlorure de sulfonyle est la même que celle dudit sulfonamide.
La présente invention vise aussi le cas où la partie organique dudit chlorure de sulfonyle porte, au moins transitoirement sous la forme d'un intermédiaire réactionnel, la fonction sulfonamide. Cela qui permet de faire des produits cycliques ou des produits polymériques. Comme cela est bien connu d'une manière générale par l'homme de métier pour les réactions de condensation, il y a cyclisation ou polycondensation selon le nombre de maillons séparant les deux fonctions et selon la dilution du mélange réactionnel. (ce phénomène très connu est, inter alia, rappelé dans la demande intercalaire WO 97/23448)

La présente invention est particulièrement utile pour réaliser la condensation de la présente invention lorsque les parties organiques, semblables ou différentes, dudit chlorure de sulfonyle et dudit sulfonamide sont choisies parmi les radicaux de formule (Rf) :

-(CX₂)ₚ-GEA

où:
· les X semblables ou différents représentent un fluor ou un radical de formule CₙF₂ₙ₊₁, avec n entier au plus égal à 5 de préférence à 2:
· p représente un entier au plus égal à 2 :
· GEA représente un groupe électroattracteur dont les éventuelles fonctions sont inertes dans les conditions de la réaction, avantageusement fluor ou un reste perfluoré de formule CₙF₂ₙ₊₁, avec n entier au plus égal à 8, avantageusement à 5.

Le nombre total de carbone de Rf est avantageusement compris entre 1 et 15, de préférence entre 1 et 10.

GEA peut être ou porter une fonction halogénure lourd de sulfonyle. Ce qui permet de préparer in situ les composés porteurs à la fois de la fonction nucléophile et de la fonction halogénure lourd de sulfonyle, composés où la partie organique dudit chlorure de sulfonyle porte la fonction sulfonamide. Cela qui permet de faire des produits cycliques ou des produits polymériques.

Comme cela a été indiqué ci-dessus, les composés les plus intéressants obtenus à partir des imides synthétisés selon la présente invention sont des dérivés lithiés, ledit procédé comporte avantageusement, après une étape éventuelle de purification et/ou d'isolement, une étape de traitement par un hydroxyde ou un sel basique de lithium.

Selon la présente invention les sulfonamides de départ peuvent être avantageusement synthétisés par action du même halogénure de sulfonyle que celui utilisé pour la synthèse de l'imide. Cette synthèse peut être réalisée dans des solvants polaires, protiques ou non, à condition qu'ils n'aient pas de propension à se faire alcoyler. En particulier la réaction peut être menée dans des éthers cycliques ou non, symétriques ou non.

Un autre but de la présente invention est de fournir un réactif utile pour les procédés du type précédent.

Ce but, et d'autres qui apparaîtront par la suite, est atteint au moyen d'un réactif qui comporte pour addition successive ou simultanée :
• un halogénure lourd (c'est-à-dire dont le nombre atomique est au moins égal à celui du chlore) de sulfonyle, avantageusement chlorure de sulfonyle et :
• une base organique à la fois non alcoylable et liposoluble :
· avantageusement un solvant peu polaire :
et par le fait que la partie organique dudit sulfonyle est perfluorée sur le carbone porté par le soufre.

Un autre but de la présente invention est de proposer l'utilisation de chlorure de sulfonyle perfluoré sur le carbone porté, par le soufre, c'est-à-dire le jouxtant pour la mono et la bissulfonylation d'ammoniaque, d'ammoniac, d'amide, y compris sulfamide. Et ce avantageusement en présence d'un base organique non alcoylable et liposoluble. Et ce notamment dans les synthèses conduisant à des sels, avantageusement alcalins, d'imide portant au moins un, de préférence deux sulfonées perfluorés sur le carbone aliphatique (c'est-à-dire sp3) jouxtant le soufre.

Un des avantages de l'utilisation du chlorure est une faible sensibilité aux réactions parasite avec l'eau.
Afin de mieux comprendre l'invention on peut donner à titre indicatifs les réactions types

Rf-SO₂Cl + NuH + B **-------->** Rf-SO₂Nu + BHCl

ou

Rf-SO₂Cl + Nu⁻ ----> + Cl⁻ + Rf-SO₂Nu

et si, comme cela est préféré, Nu est de nature Nu'H (ou plus exactement si la fonction nucléophile considérée de Nu est de structure -NH- [c'est-à-dire de structure NH- ou NH₂])

Rf-SO₂Nu'H + B ----> Rf-SO₂Nu'⁻,BH⁺

NuH et Nu⁻ représentent respectivement le nucléophile sous forme neutre et anionique.
les substrats les plus intéressants sont ceux où Nu est choisi parmi Ar-SO₂-NH-, surtout R-SO₂-NH-, y compris et de préférence Rf-SO₂-NH-.
Les exemples non limitatifs suivants illustrent l'invention :

### Exemple 1 : préparation du trifluorométhanesulfonamide : CF₃SO₂NH₂

### - réaction en milieu anhydre :

Dans un réacteur, on charge 15,3 g de chlorure de trifluorométhanesulfonyle en solution dans 103 g d'éther isopropylique anhydre. Le milieu est refroidi à 5°C et on ajoute lentement l'ammoniac en 2 h. Après 5 h d'agitation à 5°C, on ajoute 24,8 g d'eau pour dissoudre les sels. Le milieu est ensuite acidifié par ajout 20,7 g d'une solution aqueuse d'acide chlorhydrique à 36%.

Après un nouvel ajout de 13 g d'eau, les phases sont séparées. La phase aqueuse est lavée par 50 g d'éther isopropylique. Les phases organiques sont réunies et le solvant est éliminé sous pression réduite.

On obtient ainsi 10,15 g d'un solide blanc de trifluorométhanesulfonamide.
Fusion (Koffler) = 119°C

### - réaction en milieu aqueux :

La réaction est conduite de manière analogue en utilisant une solution aqueuse d'ammoniaque à 30%.

### Exemple 2 : préparation du bis trifluorométhylsulfinimide de lithium

Dans un réacteur, on charge 1 l de monochlorobenzène et 164,6 g de trifluorométhanesufonamide préparé selon l'exemple précédent. On ajoute ensuite à cette suspension 224,6 g de triéthylamine. La solution obtenue est refroidie à 5°C. Une solution de 187 g de chlorure de trifluorométhanesulfonyle dans 191 g de monochlorobenzène est ensuite ajoutée en 40 mn à 0-5°C.

On amène ensuite la température à 28°C. Après 4 h de réaction, le milieu réactionnel est filtré.

Le filtrat est dégazé par bullage d'azote puis lavé par 436 g d'eau. Les 2 phases organiques inférieures sont récupérées et lavées à nouveau par 507 g d'eau.

Ces phases organiques sont ensuite traitées par une solution de 46,7 g de lithine hydratée dans 216 g d'eau. Le milieu obtenu est biphasique.

La phase aqueuse est récupérée et extraite par 226 g d'éther isopropylique.

Cette dernière phase organique est évaporée sous pression réduite pour conduire à 206 g de bis trifluorométhanesulfonimide de lithium brut sous forme de solide jaunâtre.
Rdt = 60 %
RMN 19F = -1,8 ppm

### Exemple 3 : Réaction en présence de diisopropyléthylamine

Dans un réacteur, on charge 11,2 g (0,079 M) de l'amide de l'acide trifluorométhanesulfonique dans 73 g de monochlorobenzène. Après addition de 20,7 g (0,16 M) de diisopropyléthylamine, 14,7 g (0,087 M) de chlorure de trifluorométhanesulfonyle en solution dans 15 g de monochlorobenzène sont coulés à 10°C pendant 45 mn. Le milieu est ensuite agité pendant 4 h à 30°C.

Le traitement est réalisé comme dans l'exemple précédent et conduit à 20,4g de bis trifluorométhanesulfonimide de lithium de titre 98% (rdt = [(20,4/303)/.0,079]*0,98 = 83%

### Exemples 11 à 13 : rôle du solvant

Pour mieux mettre en évidence le rôle du solvants une base dont le sel présente une solubilité médiocre a été utilisée :

| Exemple N° | Réactifs | Solvant | Conditions | Résultats |
|---|---|---|---|---|
| 4 | CF₃SO₂NH₂=1,6g | CH₂Cl₂ | Addition CF₃SO₂Cl à 0°C | Conversion |
| | CF₃SO₂Cl = 1 éq. | | Réaction 13 h à 20°C | CF₃SO₂NH₂=80% |
| | NEt₃=2 éq. | | | Rendement = 43% |
| 5 | | CH₂Cl₂ | Introduction | |
| | CF₃SO₂NH₂=3,1 g | | CH₂Cl₂+CF₃SO₂NH₂+1 éq. NEt₃ | Conversion |
| | CF₃SO₂Cl = 1 éq. | | Addition CF₃SO₂Cl à 0°C | CF₃SO₂NH₂=76% |
| | NEt₃=2 éq. | | Coulée 1 éq. NEt₃ à 0°C | Rendement = 31% |
| | | | Réaction 3 h à 20°C | |
| 6 | CF₃SO₂NH₂=1,4 g | DIPE | Addition CF₃SO₂Cl à 0°C | Conversion |
| | CF₃SO₂Cl = 1 éq. | | Réaction 3 h à 20°C | CF₃SO₂NH₂=80% |
| | NEt₃=2 éq. | | | Rendement = 33% |
| 7 | CF₃SO₂NH₂=1 g | MCB | Addition CF₃SO₂Cl à 0°C | |
| | CF₃SO₂Cl = 1 éq. | | Réaction 13 h à 20°C | Rendement dosé = |
| | NEt₃=2 éq. | | | 67% |

| | | | | |
|---|---|---|---|---|
| DIPE : düsopropyléther | | | | |
| MCB : monochlorobenzène | | | | |

| Exemple N° | Réactifs | Solvant | Conditions | Résultats |
|---|---|---|---|---|
| 8 COMPARATIF | CF₃SO₂NH₂=1,85g | CH₂Cl₂ | Addition CF₃SO₂Cl à 0°C | Conversion |
| | CF₃SO₂Cl = 1 éq. | | Réaction 7 h à 30°C | CF₃SO₂NH =35% |
| | pyridine = 2 éq. | | Pression autogène | Rendement = 24% |
| 9 COMPARATIF | CF₃SO₂NH₂=1,62 g | DIPE | Addition CF₃SO₂Cl à 0°C | Conversion |
| | CF₃SO₂Cl = 1,1 éq. | | Réaction 7 h à 30°C | CF₃SO₂NH₂=30% |
| | pyridine = 2éq. | | Pression autogène | Rendement = 3% |
| 10 COMPARATIF | CF₃SO₂NH₂=1,02 g | MCB | Addition CF₃SO₂Cl à 0°C | Conversion |
| | CF₃SO₂Cl = 1,1 éq. | | Réaction 7 h à 30°C | CF₃SO₂NH₂=63% |
| | pyridine = 2 éq. | | Pression autogène | Rendement = 4% |
| 11 | CF₃SO₂NH₂=1,91 g | CH₂Cl₂ | Addition CF₃SO₂Cl à 0°C | Conversion |
| | CF₃SO₂Cl = 1,1 éq. | | Réaction 7 h à 20°C | CF₃SO₂NH₂=92% |
| | DIPEA=2 éq. | | | Rendement = 85% |
| 12 | CF₃SO₂NH₂=1,28 g | IPE | Addition CF₃SO₂Cl à 0°C | Conversion |
| | CF₃SO₂Cl = 1,2 éq. | | Réaction 8 h à 20°C | CF₃SO₂NH₂>91% |
| | DIPEA=2 éq. | | | Rendement = 90% |
| 13 | CF₃SO₂NH₂=1,2g | MCB | Addition CF₃SO₂Cl à 0°C | Conversion |
| | CF₃SO₂Cl = 1,2 éq. | | Réaction 8h à 20°C | CF₃SO₂NH₂> 98% |
| | DIPEA=2éq. | | | Rendement =97% |

| | | | | |
|---|---|---|---|---|
| DIPE: diisopropyléther | | | | |
| MCB : monochlorobenzène | | | | |
| DIPEA: di isopropyléthylamine | | | | |
| ** Les analyses des différents essais ont été effectuées en spectroscopie RMN du fluor avec étalon interne.* | | | | |

## Revendications

1. Procédé de sulfonylation, **caractérisé par le fait qu'**il comprend une étape de mise en contact d'un nucléophile dont l'atome nucléophile est un azote avec un réactif comportant pour addition successive ou simultanée :
· un chlorure de sulfonyle et :
· une base organique à la fois non alcoylable et liposoluble ;
**par le fait que** la partie organique dudit sulfonyle est perfluorée sur le carbone porté par le soufre et **par le fait que** ladite base organique à la fois non alcoylable et liposoluble est choisie parmi les parmi les dialcoylphosphines encombrées, trialcoylphosphines, les hydroxydes de phosphonium, les dialcoylamines encombrées, trialcoylamines, les hydroxydes d'ammonium, avec la condition que lorsque quand la réaction est menée sans un solvant, ce dernier est choisi parmi ceux dont la polarité (E^{f}t exprimée en kcal par mol) est au plus égale à 40 et avec la condition que quand la réaction est menée sans solvant, les bases organiques sont choisies parmi les trialcoylamines de nombre de carbones supérieur à 6 ; les trialcoylamines de nombre de carbone inférieur à 7, mais présentant au moins un radical secondaire ou tertiaire, avantageusement deux.

2. Procédé selon la revendication 1, **caractérisé par le fait que** ledit nucléophile, sous forme neutre ou anionique a comme acide associé un acide dont le pKa est au plus égal à environ 7, avantageusement à 6, de préférence à 5.

3. Procédé selon les revendications 1 et 2, **caractérisé par le fait que** ledit azote dudit nucléophile est relié à un groupe électroattracteur tel que sulfonyle, avantageusement choisi parmi les sulfamides des acides sulfoniques dont le soufre est lié à un aryle ou un reste aliphatique y compris les alcoyles, de préférence à un reste aliphatique perfluorés sur le carbone jouxtant le soufre.

4. Procédé selon les revendications 1 à 3, **caractérisé par le fait que** ledit nucléophile est un sel de sulfonamide (sulfamide) et d'une base organique non alcoylable.

5. Procédé selon les revendications 1 à 4, **caractérisé par le fait que** ledit nucléophile est un sulfonamide (sulfamide) sous la forme d'un sel liposoluble avec ladite base organique à la fois non alcoylable et liposoluble.

6. Procédé selon les revendications 1 à 5, **caractérisé par le fait que** la somme des bases (base du sel substrat et base organique liposoluble non alcoylable) présente un excès par rapport à la somme de l'acidité dégagée sous forme d'acide halohydrique et de l'acidité des composés sulfonés en cours de formation au plus égale à trois fois, avantageusement à deux fois, de préférence à une fois, la quantité d'acide halohydrique dégagée.

7. Procédé selon les revendications 1 à 6, **caractérisé par le fait que** ladite base, à la fois non alcoylable et liposoluble, présente au moins une solubilité dans le benzène significative (symbole "s" dans le "handbook of chemistry and physics"), avantageusement (symbole "v" dans le "handbook of chemistry and physics") élevée.

8. Procédé selon les revendications 1 à 7, **caractérisé par le fait que** ladite base organique présente au moins une solubilité dans le benzène significative (symbole "s" dans le "handbook of chemistry and physics"), avantageusement (symbole "v" dans le "handbook of chemistry and physics") élevée.

9. Procédé selon les revendications 4 à 8, **caractérisé par le fait que** le pKa de l'acide associé à ladite base organique non alcoylable est supérieur ou égal à celui dudit sulfonamide.

10. Procédé selon les revendications 1 à 9, **caractérisé par le fait que** le pKa de l'acide associé à ladite base organique non alcoylable et liposoluble est supérieur ou égal à celui dudit sulfonamide.

11. Procédé selon les revendications 1 à 10, **caractérisé par le fait que** lorsque ledit nucléophile est un sel de sulfonamide (sulfamide) et d'une base organique non alcoylable, ladite base organique non alcoylable et ladite base à la fois non alcoylable et liposoluble sont identiques.

12. Procédé selon les revendications 1 à 11, **caractérisé par le fait que** ladite mise en contact est réalisée dans un solvant organique, avantageusement peu polaire, de préférence peu miscible à l'eau (au plus 10% en masse, avantageusement au plus 5% en masse, de préférence au plus 2% en masse).

13. Procédé selon les revendications 1 à 12, **caractérisé par le fait que** ladite mise en contact est réalisée dans un solvant organique, lequel est choisi de manière que ledit sel y soit soluble, avantageusement à hauteur d'une concentration d'au moins 0,05 M, de préférence d'au moins 0,2 M.

14. Procédé selon les revendications 1 à 13, **caractérisé par le fait que** la quantité de ladite base non alcoylable et liposoluble mise en jeu au cours de la réaction est au moins égale à la quantité nécessaire pour neutraliser l'acide halohydrique dégagé.

15. Procédé selon les revendications 1 à 14, **caractérisé par le fait que** la réaction est menée à une température d'au plus 100°C, avantageusement à 50°C, de préférence à 40°C.

16. Procédé selon les revendications 1 à 15, **caractérisé par le fait que** la partie organique dudit chlorure de sulfonyle est la même que celle dudit sulfonamide.

17. Procédé selon les revendications 1 à 16, **caractérisé par le fait que** les parties organiques, semblables ou différentes dudit chlorure de sulfonyle et dudit sulfonamide, sont choisies parmi les radicaux de formule (Rf):
-(CX₂)ₚ-GEA
où :
· les X, semblables ou différents, représentent un fluor ou un radical de formule CₙF₂ₙ₊₁, avec n entier au plus égal à 5 de préférence à 2 ;
· p représente un entier au plus égal à 2 ;
· GEA représente un groupe électroattracteur dont les éventuelles fonctions sont inertes dans les conditions de la réaction, avantageusement fluor ou un reste perfluoré de formule CₙF₂ₙ₊₁, avec n entier au plus égal à 8, avantageusement à 5.
Le nombre total de carbone de Rf est avantageusement compris entre 1 et 15, de préférence entre 1 et 10.

18. Procédé selon les revendications 1 à 17, **caractérisé par le fait que** ledit nucléophile dont l'atome nucléophile est un azote, est un sel de sulfonamide (sulfamide) et d'une base organique non alcoylable, et **par le fait que** ledit procédé comporte après une étape éventuelle de purification et/ou d'isolement, une étape de traitement par un hydroxyde ou un sel basique de lithium.

19. Réactif utile pour la mise en oeuvre du procédé selon les revendications 1 à 18, **caractérisé par le fait qu'**il comporte pour addition successive ou simultanée :
· un halogénure lourd (c'est-à-dire dont le nombre atomique est au moins égal à celui du chlore) de sulfonyle, avantageusement chlorure de sulfonyle, et :
· une base organique à la fois non alcoylable et liposoluble choisie parmi les parmi les dialcoylphosphines encombrées, trialcoylphosphines, les hydroxydes de phosphonium, les dialcoylamines encombrées, trialcoylamines, les hydroxydes d'ammonium ;
· un solvant peu polaire choisi parmi ceux dont la polarité (E^{f}ₜ exprimée en kcal par mol) est au plus égale à 40 ;
et **par le fait que** la partie organique dudit sulfonyle est perfluorée sur le carbone porté par le soufre.

20. Réactif selon la revendication 19, **caractérisé par le fait que** ledit solvant, y compris mélange de solvant, peu polaire est choisi parmi ceux présentant une faible solubilité dans l'eau, et ne présentant pas une chaîne aliphatique chlorée.

21. Réactif selon les revendications 19 et 20, **caractérisé par le fait que** ledit solvant peu polaire est choisi parmi les composés organiques oxygénés, (notamment les éthers, les esters, voire les cétones), les hydrocarbures (y compris fractions pétrolières), les hydrocarbures aromatiques halogénés sur le noyau.

22. Réactif selon les revendications 19 à 21, **caractérisé par le fait que** ledit solvant peu polaire est choisi parmi les benzènes substitués et les hydrocarbures halogénés sur le noyau.

## Claims

1. Sulphonylation process, **characterized in that** it comprises a stage in which a nucleophile, the nucleophilic atom of which is a nitrogen, is brought into contact with a reactant comprising, for successive or simultaneous addition:
• a sulphonyl chloride, and;
• an organic base which simultaneously cannot be alkylated and is liposoluble;
**in that** the organic part of the said sulphonyl is perfluorinated on the carbon carried by the sulphur and **in that** the said organic base which simultaneously cannot be alkylated and is liposoluble is chosen from hindered dialkylphosphines, trialkylphosphines, phosphonium hydroxides, hindered dialkylamines, trialkylamines or ammonium hydroxides, with the condition that, when the reaction is carried out in a solvent, the latter is chosen from those for which the polarity (E^{f}ₜ, expressed in kcal per mol) is at most equal to 40 and with the condition that, when the reaction is carried out without solvent, the organic bases are chosen from trialkylamines whith a carbon number of greater than 6; trialkylamines with a carbon number of less than 7, but exhibiting at least one secondary or tertiary radical, advantageously two.

2. Process according to Claim 1, **characterized in that** the said nucleophile, in the neutral or anionic form, has, as associated acid, an acid for which the pKₐ is at most equal to approximately 7, advantageously to 6, preferably to 5.

3. Process according to Claims 1 and 2, **characterized in that** the said nitrogen of the said nucleophile is connected to an electron-withdrawing group, such as a sulphonyl group, advantageously chosen from sulphamides of sulphonic acids in which the sulphur is bonded to an aryl or an aliphatic residue, including alkyls, preferably to an aliphatic residue perfluorinated on the carbon adjoining the sulphur.

4. Process according to Claims 1 to 3, **characterized in that** the said nucleophile is a salt of sulphonamide (sulphamide) and of an organic base which cannot be alkylated.

5. Process according to Claims 1 to 4, **characterized in that** the said nucleophile is a sulphonamide (sulphamide) in the form of a liposoluble salt with the said organic base which simultaneously cannot be alkylated and is liposoluble.

6. Process according to Claims 1 to 5, **characterized in that** the sum of the bases (base of the substrate salt and liposoluble organic base which cannot be alkylated) exhibits an excess with respect to the sum of the acidity given off in the form of hydrohalic acid and of the acidity of the sulphonated compounds being formed at most equal to three times, advantageously to two times, preferably to one times, the amount of hydrohalic acid given off.

7. Process according to Claims 1 to 6, **characterized in that** the said base which simultaneously cannot be alkylated and is liposoluble exhibits at least a significant (symbol "s" in the Handbook of Chemistry and Physics) solubility in benzene, advantageously a high (symbol "v" in the Handbook of Chemistry and Physics) solubility in benzene.

8. Process according to Claims 1 to 7, **characterized in that** the said organic base exhibits at least a significant (symbol "s" in the Handbook of Chemistry and Physics) solubility in benzene, advantageously a high (symbol "v" in the Handbook of Chemistry and Physics) solubility in benzene.

9. Process according to Claims 4 to 8, **characterized in that** the pKₐ of the acid associated with the said organic base which cannot be alkylated is greater than or equal to that of the said sulphonamide.

10. Process according to Claims 1 to 9, **characterized in that** the pKₐ of the acid associated with the said organic base which cannot be alkylated and is liposoluble is greater than or equal to that of the said sulphonamide.

11. Process according to Claims 1 to 10, **characterized in that**, when the said nucleophile is a salt of sulphonamide (sulphamide) and of an organic base which cannot be alkylated, the said organic base which cannot be alkylated and the said base which simultaneously cannot be alkylated and is liposoluble are identical.

12. Process according to Claims 1 to 11, **characterized in that** the said operation of bringing into contact is carried out in an organic solvent, advantageously of low polarity, preferably of low miscibility with water (at most 10% by mass, advantageously at most 5% by mass, preferably at most 2% by mass).

13. Process according to Claims 1 to 12, **characterized in that** the said operation of bringing into contact is carried out in an organic solvent which is chosen so that the said salt is soluble therein, advantageously to a concentration level of at least 0.05M, preferably of at least 0.2M.

14. Process according to Claims 1 to 13, **characterized in that** the amount of the said base which cannot be alkylated and is liposoluble introduced during the reaction is at least equal to the amount necessary to neutralize the hydrohalic acid given off.

15. Process according to Claims 1 to 14, **characterized in that** the reaction is carried out at a temperature of at most 100°C, advantageously at 50°C, preferably at 40°C.

16. Process according to Claims 1 to 15, **characterized in that** the organic part of the said sulphonyl chloride is the same as that of the said sulphonamide.

17. Process according to Claims 1 to 16, **characterized in that** the organic parts, which are alike or different, of the said sulphonyl chloride and of the said sulphonamide are chosen from radicals of formula (Rf):
-(CX₂)ₚ-EWG
where:
· the X groups, which are alike or different, represent a fluorine or a radical of formula CₙF₂ₙ₊₁, with n an integer at most equal to 5, preferably to 2;
· p represents an integer at most equal to 2;
· EWG represents an electron-withdrawing group, the possible functional groups of which are inert under the reaction conditions, advantageously fluorine or a perfluorinated residue of formula CₙF₂ₙ₊₁, with n an integer at most equal to 8, advantageously to 5.
The total carbon number of Rf is advantageously between 1 and 15, preferably between 1 and 10.

18. Process according to Claims 1 to 17, **characterized in that** the said nucleophile in which the nucleophilic atom is a nitrogen is a salt of sulphonamide (sulphamide) and of an organic base which cannot be alkylated and **in that** the said process comprises, after an optional purification and/or isolation stage, a stage of treatment with lithium hydroxide or a basic lithium salt.

19. Reactant of use in the implementation of the process according to Claims 1 to 18, **characterized in that** it comprises, for successive or simultaneous addition:
· a sulphonyl heavy halide (that is to say, in which the atomic number of the halogen is at least equal to that of chlorine), advantageously sulphonyl chloride, and;
· an organic base which simultaneously cannot be alkylated and is liposoluble, chosen from hindered dialkylphosphines, trialkylphosphines, phosphonium hydroxides, hindered dialkylamines, trialkylamines or ammonium hydroxides;
· a solvent of low polarity chosen from those for which the polarity (E^{f}ₜ, expressed in kcal per mol) is at most equal to 40;
and **in that** the organic part of the said sulphonyl is perfluorinated on the carbon carried by the sulphur.

20. Reactant according to Claim 19, **characterized in that** the said solvent, including mixture of solvents, of low polarity is chosen from those exhibiting a low solubility in water and not exhibiting a chlorinated aliphatic chain.

21. Reactant according to Claims 19 and 20, **characterized in that** the said solvent of low polarity is chosen from oxygen-containing organic compounds (in particular ethers, esters, even ketones), hydrocarbons (including petroleum fractions) or ring-halogenated aromatic hydrocarbons.

22. Reactant according to Claims 19 to 21, **characterized in that** the said solvent of low polarity is chosen from substituted benzenes and ring-halogenated aromatic hydrocarbons.

## Patentansprüche

1. Verfahren zur Sulfonylierung, **dadurch gekennzeichnet, daß** es einen Schritt des Inkontaktbringens eines Nucleophils, dessen nucleophiles Atom ein Stickstoff ist, mit einem Reagenz, welches für die sukzessive oder gleichzeitige Zugabe
• ein Sulfonylchlorid und
• eine gleichzeitig nichtalkylierbare und fettlösliche organische Base
aufweist, umfaßt, daß der organische Teil des Sulfonyls an dem vom Schwefel getragenen Kohlenstoff perfluoriert ist und daß die gleichzeitig nichtalkylierbare und fettlösliche organische Base ausgewählt ist aus gehinderten Dialkylphosphinen, Trialkylphosphinen, Phosphoniumhydroxiden, gehinderten Dialkylaminen, Trialkylaminen und Ammoniumhydroxiden, jedoch mit der Maßgabe, daß, wenn die Reaktion ohne Lösemittel durchgeführt wird, letztgenannte Verbindung ausgewählt wird aus solchen Verbindungen, deren Polarität (E^{f}_{t,} berechnet in kcal pro mol) höchstens 40 beträgt, und mit der Maßgabe, daß, wenn die Reaktion ohne Lösemittel durchgeführt wird, die organischen Basen ausgewählt sind aus Trialkylaminen mit einer Kohlenstoffanzahl von größer 6 und Trialkylaminen mit einer Kohlenstoffzahl kleiner 7, jedoch mit mindestens einem, vorzugsweise zwei sekundären oder tertiären Resten.

2. Verfahren nach Anspruch 1, **dadurch gekennzeichnet, daß** das Nucleophil, in neutraler oder anionischer Form, als gebundene bzw. assoziierte Säure eine Säure aufweist, deren pKa-Wert höchstens etwa 7, vorzugsweise 6, bevorzugt 5, beträgt.

3. Verfahren nach Anspruch 1 und 2, **dadurch gekennzeichnet, daß** der Stickstoff des Nucleophils mit einer elektronenziehenden Gruppe verbunden ist, wie Sulfonyl, vorzugsweise ausgewählt aus Sulfamiden von Sulfonsäuren, deren Schwefel an ein Aryl oder einen aliphatischen Rest einschließlich Alkylen gebunden ist, vorzugsweise an einen am zum Schwefel benachbarten Kohlenstoff perfluorierten Alkylrest.

4. Verfahren nach den Ansprüchen 1 bis 3, **dadurch gekennzeichnet, daß** das Nucleophil ein Salz eines Sulfonamids (Sulfamids) und einer nichtalkylierbaren organischen Base ist.

5. Verfahren nach den Ansprüchen 1 bis 4, **dadurch gekennzeichnet, daß** das Nucleophil ein Sulfonamid (Sulfamid) in Form eines fettlöslichen Salzes mit der gleichzeitig nichtalkylierbaren und fettlöslichen organischen Base ist.

6. Verfahren nach den Ansprüchen 1 bis 5, **dadurch gekennzeichnet, daß** die Summe der Basen (Base des Ausgangssalzes und nichtalkylierbare fettlösliche organische Base) einen Überschuß in bezug auf die Summe der eingesetzten Acidität in Form einer Halogenwasserstoffsäure und der Acidität der im Verlauf der Bildung sulfonierten Verbindungen von höchstens dem Dreifachen, vorzugsweise dem Zweifachen, vorzugsweise dem Einfachen, der eingesetzten Halogenwasserstoffsäuremenge darstellt.

7. Verfahren nach den Ansprüchen 1 bis 6, **dadurch gekennzeichnet, daß** die gleichzeitig nichtalkylierbare und fettlösliche Base eine in Benzol bedeutsame bzw. signifikante Löslichkeit (Symbol "*s*" im *"Handbook of Chemistry and Physics"*), vorzugsweise eine hohe Löslichkeit (Symbol "*v*" im *"Handbook of Chemistry and Physics"*), aufweist.

8. Verfahren nach den Ansprüchen 1 bis 7, **dadurch gekennzeichnet, daß** die organische Base eine in Benzol bedeutsame bzw. signifikante Löslichkeit (Symbol "*s*" im *"Handbook of Chemistry and Physics"*), vorzugsweise eine hohe Löslichkeit (Symbol "*v*" im *"Handbook of Chemistry and Physics"*), aufweist.

9. Verfahren nach den Ansprüchen 4 bis 8, **dadurch gekennzeichnet, daß** der pKa-Wert der mit der nichtalkylierbaren organischen Base gebundenen bzw. assoziierten Säure größer oder gleich dem pKa-Wert des Sulfonamids ist.

10. Verfahren nach den Ansprüchen 1 bis 9, **dadurch gekennzeichnet, daß** der pKa-Wert der mit der nichtalkylierbaren und fettlöslichen organischen Base gebundenen bzw. assoziierten Säure größer oder gleich dem pKa-Wert des Sulfonamids ist.

11. Verfahren nach den Ansprüchen 1 bis 10, **dadurch gekennzeichnet, daß**, wenn das Nucleophil ein Salz eines Sulfonamids (Sulfamids) und einer nichtalkylierbaren organischen Base ist, die nichtalkylierbare organische Base und die gleichzeitig nichtalkylierbare und fettlösliche Base identisch sind.

12. Verfahren nach den Ansprüchen 1 bis 11, **dadurch gekennzeichnet, daß** das Inkontaktbringen in einem organischen, vorzugsweise wenig polarem, bevorzugt wenig mit Wasser mischbaren (höchstens zu 10 Masse-%, vorzugsweise höchstens zu 5 Masse-%, bevorzugt höchstens zu 2 Masse-%) Lösemittel erfolgt.

13. Verfahren nach den Ansprüchen 1 bis 12, **dadurch gekennzeichnet, daß** das Inkontaktbringen in einem organischen Lösemittel durchgeführt wird, welches derart ausgewählt ist, daß das Salz darin löslich ist, vorzugsweise mit einer Konzentrationsmenge von mindestens 0.05 M, vorzugsweise mindestens 0.2 M.

14. Verfahren nach den Ansprüchen 1 bis 13, **dadurch gekennzeichnet, daß** die Menge der im Verlauf der Reaktion eingesetzten, nichtalkylierbaren und fettlöslichen Base mindestens der für die Neutralisation der eingesetzten Halogenwasserstoffsäure erforderlichen Menge entspricht.

15. Verfahren nach den Ansprlichen 1 bis 14, **dadurch gekennzeichnet, daß** die Reaktion bei einer Temperatur von höchstens 100 °C, vorzugsweise bei 50°C, bevorzugt bei 40 °C, durchgeführt wird.

16. Verfahren nach den Ansprüchen 1 bis 15, **dadurch gekennzeichnet, daß** der organische Teil des Sulfonylchlorids derselbe wie der organische Teil des Sulfonamids ist.

17. Verfahren nach den Ansprüchen 1 bis 16, **dadurch gekennzeichnet, daß** die organischen Teile, gleich oder verschieden, des Sulfonylchlorids und des Sulfonamids ausgewählt sind aus Resten der Formel (Rf)
-(CX₂)ₚ-GEA
wobei:
• X, gleich oder verschieden, Fluor oder einen Rest der Formel CₙF_{2n-1,} wobei n eine ganze Zähl von höchstens 5, vorzugsweise 2, ist, darstellt;
• p eine ganze Zahl von höchstens 2 ist;
• GEA eine elektronenziehende Gruppe, deren gegebenenfalls vorhandenen Funktionen unter den Reaktionsbedingungen inert sind, vorzugsweise Fluor oder einen perfluorierten Rest der Formel CₙF₂ₙ₋₁, wobei n eine ganze Zahl von höchstens 8, vorzugsweise 5, ist, darstellt;
wobei die Gesamtanzahl an Kohlenstoffen von Rf vorzugsweise zwischen 1 und 15, bevorzugt zwischen 1 und 10, liegt.

18. Verfahren nach den Ansprüchen 1 bis 17, **dadurch gekennzeichnet, daß** das Nucleophil, dessen nucleophiles Atom ein Stickstoff ist, ein Salz eines Sulfonamids (Sulfamids) und einer nichtalkylierbaren organischen Base ist und daß das Verfahren nach einem optionalen Verfahrensschritt der Reinigung und/oder der Isolierung einen Verfahrensschritt der Behandlung mit einem Hydroxid oder einem basischen Lithiumsalz umfaßt.

19. Reagenz für den Einsatz in dem Verfahren nach den Ansprüchen 1 bis 18, **dadurch gekennzeichnet, daß** es für die sukzessive oder gleichzeitige Zugabe
• ein Sulfonyl eines schweren Halogenids (d. h. eines Halogenids, dessen Atomzahl wenigstens der Atomzahl des Chlors beträgt), vorzugsweise Sulfonylchlorid, und
• eine gleichzeitig nichtalkylierbare und fettlösliche organische Base, ausgewählt aus gehinderten Dialkylphosphinen. Trialkylphosphinen, Phosphoniumhydroxyden, gehinderten Dialkylaminen, Trialkylaminen und Ammoniumhydroxyden; sowie
• ein wenig polares Lösemittel, ausgewählt aus Lösemitteln mit einer Polarität (E^{f}ₜ, berechnet in kcal pro mol) höchstens 40 beträgt;
aufweist und daß der organische Teil des Sulfonyls an dem vom Schwefel getragenen Kohlenstoff perfluoriert ist.

20. Reagenz nach Anspruch 19, **dadurch gekennzeichnet, daß** das wenig polare Lösemittel einschließlich einer Lösemittelmischung ausgewählt ist aus solchen Lösemitteln mit einer geringen Wasserlöslichkeit und ohne chlorierte aliphatische Kette.

21. Reagenz nach den Ansprüchen 19 und 20, **dadurch gekennzeichnet, daß** das wenig polare Lösemittel ausgewählt ist aus oxygenierten organischen Verbindungen (insbesondere Ethern, Estern oder Ketonen), Kohlenwasserstoffen (einschließlich Petrolfraktionen) und am Kern halogenierten aromatischen Kohlenwasserstoffen.

22. Reagenz nach den Ansprüchen 19 bis 21, **dadurch gekennzeichnet, daß** das wenig polare Lösemittel ausgewählt ist aus substituiertem Benzolen und am Kern halogenierten Kohlenwasserstoffen.
